Europäisches Patentamt

European Patent Office

Office européen des brevets

(19)

(11) **EP 0 742 023 B1**

(12) **EUROPEAN PATENT SPECIFICATION**

(45) Date of publication and mention
of the grant of the patent:
**22.01.2003 Bulletin 2003/04**

(51) Int Cl.$^7$: **A61L 31/04**, A61L 31/14

(21) Application number: **96400997.1**

(22) Date of filing: **09.05.1996**

(54) **Biocompatible material**

Biovertragliches Material

Matériau biocompatible

(84) Designated Contracting States:
**BE DE ES FR GB IT NL**

(30) Priority: **09.05.1995 JP 13600495**

(43) Date of publication of application:
**13.11.1996 Bulletin 1996/46**

(73) Proprietors:
- **Terano, Minoru**
  **Nomi-gun, Ishikawa-ken (JP)**
- **Yui, Nobuhiko**
  **Nomi-gun, Ishikawa-ken (JP)**
- **Chisso Corporation**
  **Osaka-shi Osaka 530-0055 (JP)**

(72) Inventors:
- **Terano, Minoru, University Dormitory A-35**
  **Nomi-gun, Ishikawa-ken (JP)**
- **Yui, Nobuhiko, University Dormitory A-11**
  **Nomi-gun, Ishikawa-ken (JP)**

(74) Representative: **Portal, Gérard et al**
**Cabinet Beau de Loménie**
**158, rue de l'Université**
**75340 Paris Cédex 07 (FR)**

(56) References cited:
**EP-A- 0 703 253          US-A- 4 873 292**

- **DATABASE WPI Section Ch, Week 9506 Derwent Publications Ltd., London, GB; Class A17, AN 95-040351 XP002065799 & JP 06 319 784 A (TERUMO CORP)**
- **DATABASE WPI Section Ch, Week 8746 Derwent Publications Ltd., London, GB; Class A26, AN 87-325649 XP002065800 & JP 62 233 165 A (SHINGIJUTSU KAIHATSU KK)**
- **DATABASE WPI Section Ch, Week 9205 Derwent Publications Ltd., London, GB; Class A17, AN 92-036268 XP002065801 & JP 03 281 647 A (TERUMO CORP)**

**Description**

The present invention relates to a biocompatible material, and more particularly to a polyolefin-based polymeric biocompatible material for medical uses which has a surface having a superior blood anti-coagulating property.

It is desirable that a medical instrument such as an artificial organ, a blood bag, an intravenous catheter, or an extracorporeal circulation circuit for an artificial dialyzator or lung have a high biocompatibility, particularly a high blood compatibility (blood anti-coagulating property).

A medical instrument has often been prepared from a polyolefinic resin such as a polypropylene or a polyethylene because of, for example, its high mechanical strength and moldability. More specifically, a polyethylene and a polypropylene are utilized in the manufacture of a disposable medical instrument such as a syringe, a bag or a tube. (See for instance document JP06319784 A (TERUMO CORP = database WPI Section Ch Week 9506, Derwent publications abstract XP 002065799).

Document JP 62-233165 A = database WPI Section Ch Week 8746, Derwent publications ltd, abstract XP 002065800 discloses an anti-thrombus medical circuit having a surface made of a specified polyamide.

JP 03 281 647 A ( TERUMO CORP) = database WPI Section Ch Week 9205, Derwent publications ltd, abstract XP 002065801 discloses a radiation resistant polypropylene composition for a medical instrument which shows high impact strength, after radiation, high clarity and good processability at high speed moulding.

Document EP-A-0703253 (JAPAN RESEARCH DEVELOPMENT CORP) is an intermediate document having a publication date of March 27, 1996 which discloses an olefin block copolymer and a process for producing the same. The material described may exhibit a microdomain structure formed of hard segments (crystalline regions) and soft segments (amorphous regions) but provides no information how to control the microdomain structure contrary to the present invention as defined in the claims.

To impart biocompatibility to a medical instrument made of such a polyolefinic resin, chemical surface treatment has been effected on that surface of the medical instrument which contacts blood. Such a chemical surface treatment typically includes fixation of an anti-thrombus agent such as heparin on the blood contacting surface. However, this treatment must be conducted on every medical instrument and is laborious. In addition, the anti-thrombus effect is not sufficient, and may be lowered due to peeling of the anti-thrombus agent off the surface.

Accordingly, it is an object of the present invention to provide a biocompatible polymeric material which has a practically superior biocompatibility, particularly blood compatibility (anti-coagulating property) without being subjected to chemical surface treatment, and which is suitable for use in various medical instruments.

Another object of the invention is to provide a medical instrument made from such a biocompatible material.

These and other objects which will be apparent from the following detailed description have been attained according to the present invention by a biocompatible material as defined in claim 1.

Further, according to the present invention, there is provided a medical instrument having a portion to contact blood, wherein the blood contacting portion is made of the biocompatible material of the invention.

This invention can be more fully understood from the following detailed description when taken in conjunction with the accompanying drawings, in which:

FIG. 1 shows an extracorporeal blood circulation circuit which is an example of a medical instrument according to the present invention;
FIG. 2 shows a blood bag which is another example of a medical instrument according to the present invention; and
FIG. 3 shows an intravenous catheter which is a still another example of a medical instrument according to the present invention.

In general, plasma proteins such as albumin and fibrinogen are adsorbed on the surface of a material which contacts blood, and these proteins change in their secondary structure due to the adsorption. The change of the secondary structure promotes further adsorption of the plasma proteins, resulting in the formation of multiple protein adsorption layers. The multiple adsorption protein layers activate the blood platelets which contact the protein layers, eventually coagulating the blood.

The present inventors have studied on materials which can suppress the multiple adsorption of plasma proteins, thereby preventing the activation of blood platelets. As a result, the present inventors have found that the desired objects can be attained by using a specified polymer, and making the surface region of the polymer into a micro-phase separation crystalline structure.

The biocompatible material of the invention is made of a polymeric material having repeating units derived from at least one monomer selected from the group consisting of olefin monomers having 2 to 10 carbon atoms and diene monomers having 4 to 15 carbon atoms. The polymeric material includes a homopolymer and a copolymer, and the copolymer includes random and block copolymers. These polymers may be used singly or in combination of two or more thereof.

Specific examples of the homopolymer include an ethylene homopolymer, a propylene homopolymer and a butene-1 homopolymer. Specific examples of the copolymer include an ethylene-propylene copolymer, an ethylene-propylene-butene-1 copolymer, an ethylene-hexene-1 copolymer, an ethylene-octene-1 copolymer, an ethylene-butene-1-hexene-1 copolymer, an ethylene-butene-1-octene-1 copolymer, an ethylene-hexene-1-octene-1 copolymer, and an ethylene-butene-1-hexene-1-octene-1 copolymer. Of these polymers, an ethylene homopolymer, a propylene homopolymer, an ethylene-propylene copolymer, and an ethylene-propylene-butene-1 copolymer are most preferred. In particular, when a molded product having a high impact strength, a high flexibility, and a high transparency is desired, an ethylene-propylene copolymer, and an ethylene-propylene-butene-1 copolymer are preferably used.

The polymers described above can be produced by any suitable method well known in the art. For example, the predetermined monomer or monomers may be polymerized in the presence of a polymerizing catalyst comprising a combination of transition metal compound catalyst such as titanium tetrachloride with a cocatalyst comprising an organometallic compound such as an organoaluminum compound.

Generally, polymeric materials may be classified, from the view point of molecular stereostructure, into isotactic, syndiotactic and atactic stereostructure. Isotactic and syndiotactic polymers relatively readily crystallize, while atactic polymers are difficult to crystallize. In the present invention, the micro-phase separation structure consisting of crystalline and amorphous phases is formed in the surface of the polymeric material. To realize such a micro-phase separation structure more assuredly, polymers containing atactic portions are preferably used. The content of such atactic portion may be expressed by tacticity. The higher the tacticity, the smaller the content of the atactic portions. In the present invention, the tacticity is represented by the percentage by weight of the extraction residue remaining after extracting a piece of a polymer with a sufficiently large volume of boiling heptane for 6 hours in a Soxhlet's extractor.

The polymer used in the invention preferably has a tacticity corresponding to a heptane extraction residue of about 40 to 99% by weight, more preferably about 70 to 99% by weight, most preferably about 80 to 99% by weight. To synthesize the polymer having such a tacticity, use is preferably made of the methods disclosed in U.S. Patents 4,550,144 and 4,499,247. The tacticity is deeply related with the degree of crystallinity, and is nearly proportional to the degree of crystallinity.

The biocompatible material made of the polymers noted above according to the invention has a micro-phase separation structure constituted by crystalline and amorphous phases in at least surface region thereof. The surface region has a degree of crystallinity of about 40 to 70%. When the degree of crystallinity in the surface falls within this range, the crystalline phase and the amorphous phase can exist at a suitable balance, thereby providing a higher blood compatibility (blood anti-coagulating property). The degree of crystallinity at the surface is most preferably 50 to 60%.

In the present invention, the degree of crystallinity at the surface is obtained according to the method described in J. Appl. Polym. Sci., 2, 166 (1959) by measuring absorbances at 917 $cm^{-1}$, 972 $cm^{-1}$ and 997 $cm^{-1}$ by the ATR method using Fourier transform infrared spectrophotometer (FT-IR) VALOR-III available from JASCO CO., LTD., and substituting the obtained values into the following equation:

$$\text{Crystallinity (\% by weight)} = \frac{(A_{997} - A_{917})}{(A_{972} - A_{917})} \times 109 - 31.4$$

where $A_{997}$ is the absorbance at 997 $cm^{-1}$, $A_{972}$ is the absorbance at 972 $cm^{-1}$, and $A_{917}$ is the absorbance at 917 $cm^{-1}$. By the ATR method, the crystallinity to a depth of about 2 $\mu$m from the surface can be measured.

In the present invention, the micro-phase separation structure may be either of lamella structure or of sea-and-island structure. The sea-and-island structure may be either that in which the crystalline phase constitutes the sea and the amorphous phase constitutes the islands, or that in which the amorphous phase constitutes the sea and the crystalline phase constitutes the islands. Usually, the crystalline phase constitutes the islands of spherical form, while the amorphous phase constitutes the sea. The long period in the repeated thickness, i.e., the average distance of crystalline lamella, of such micro-phase separation structure is 8 to 20 nm. When the long period falls within this range, the material of the invention can more effectively suppress the adhesion of plasma proteins thereto. The long period is most preferably 9 to 18 nm.

In the present invention, the long period in the repeated thickness of the micro-phase separation structure is obtained by measuring X-ray diffraction peaks within 2 = 0 - 2 by the small angle X-ray scattering method, with the scattering by the air removed, using X-ray diffraction device JEOL JDX-8200T available from JOEL, LTD., Japan, and calculating by the method of elastic center according to the Bragg's formula.

Further, the crystallite thickness of the micro-phase separation structure of the invention is 4 to 14 nm. When the thickness of the crystalline phase falls within this range, the material of the invention can more effectively suppress the adhesion of plasma proteins thereto. The thickness of the crystalline phase is most preferably 10 to 12 nm.

In the present invention, the crystallite thickness is calculated as the product of the long period noted above and the degree of crystallinity of the entire biocompatible material (bulk crystallinity). The degree of bulk crystallinity of the biomedical material is obtained according to the method described in J. Appl. Polym. Sci., 2, 166 (1959) by measuring absorbances at 917 cm$^{-1}$, 972 cm$^{-1}$ and 997 cm$^{-1}$ by the KBr method using Fourier transform infrared spectrophotometer (FT-IR) VALOR-III available from JASCO CO., LTD., and substituting the obtained values into the following equation:

$$\text{Crystallinity (\% by weight)} = \frac{(A_{977} - A_{917})}{(A_{972} - A_{917})} \times 109 - 31.4$$

where $A_{997}$ is the absorbance at 997 cm$^{-1}$, $A_{972}$ is the absorbance at 972 cm$^{-1}$, and $A_{917}$ is the absorbance at 917 cm$^{-1}$.

The biocompatible material of the invention may be obtained in the form of, for example, sheet, film, tube or molded article by compression molding, injection molding, blow molding, inflation molding, T-die molding, calender molding, profile extrusion molding, vacuum sizing or the like molding method. The surface crystalline structure described above depends on various molding conditions. For example, molded articles having different surface crystallinities are obtained even starting from the same polymers when the cooling rates for the molten polymers are different. The higher the cooling rates, the lower the surface crystallinity.

More specific methods for producing the biocompatible material having the surface crystalline structure defined in the invention as above are as follows.

(1) Compression molding: A propylene homopolymer having a tacticity corresponding to a heptane extraction residue of 85 to 98% by weight and a melting temperature of 160 to 165°C, an ethylene-propylene copolymer having an ethylene content of 0.5 to 8% by weight and a melting temperature of 155 to 120C, or an ethylene-propylene-butene-1 copolymer having an ethylene content of 0.5 to 8% by weight, a butene-1 content of 0.5 to 8% by weight and a melting temperature of 155 to 120°C is melted at 180 to 250°C for 2 to 10 minutes, and press molded while being cooled at 130 to 20°C for 2 to 10 minutes to obtain a biocompatible material of the invention in the form of sheet or film having a thickness of 0.05 to 5 mm.

(2) Injection molding: A propylene homopolymer having a tacticity corresponding to a heptane extraction residue of 85 to 98% by weight and a melting temperature of 160 to 165°C, an ethylene-propylene copolymer having an ethylene content of 0.5 to 8% by weight and a melting temperature of 155 to 120°C, or an ethylene-propylene-butene-1 copolymer having an ethylene content of 0.5 to 8% by weight, a butene-1 content of 0.5 to 8% by weight and a melting temperature of 155 to 120C is melted at a cylinder temperature of 180 to 300C, injected into a mold, and cooled at a mold-cooling liquid temperature of 130 to 30C for 2 to 10 minutes to obtain a biocompatible material of the invention in the form of an article having a thickness of 0.05 to 5 mm.

(3) Blow molding: A propylene homopolymer having a tacticity corresponding to a heptane extraction residue of 85 to 98% by weight and a melting temperature of 160 to 165°C, an ethylene-propylene copolymer having an ethylene content of 0.5 to 8% by weight and a melting temperature of 155 to 120°C, or an ethylene-propylene-butene-1 copolymer having an ethylene content of 0.5 to 8% by weight, a butene-1 content of 0.5 to 8% by weight and a melting temperature of 155 to 120°C is melted in an extruder at a cylinder temperature of 180 to 300°C, extruded toward a mold, and cooled at a mold-cooling liquid temperature of 130 to 30°C and a blowing air temperature of 5 to 40°C for 0.5 to 10 minutes to obtain a biocompatible material of the invention in the form of a hollow article (tube) having a thickness of 0.5 to 3 mm.

(4) Inflation molding: A propylene homopolymer having a tacticity corresponding to a heptane extraction residue of 88 to 90% by weight and a melting temperature of 160 to 165°C, an ethylene-propylene copolymer having an ethylene content of 0.5 to 8% by weight and a melting temperature of 155 to 120°C, or an ethylene-propylene-butene-1 copolymer having an ethylene content of 0.5 to 8% by weight, a butene-1 content of 0.5 to 8% by weight and a melting temperature of 155 to 120°C is melted in an extruder at a cylinder temperature of 180 to 300°C, extruded as a tubular body, and cooled at a blowing air temperature of 5 to 40°C to prepare a biocompatible material of the invention in the form of a tubular film having a thickness of 0.01 to 0.5 mm.

(5) Vacuum sizing: A propylene homopolymer having a tacticity corresponding to a heptane extraction residue of 80 to 99% by weight and a melting temperature of 160 to 165°C, an ethylene-propylene copolymer having an ethylene content of 0.5 to 8% by weight and a melting temperature of 155 to 120°C, or an ethylene-propylene-butene-1 copolymer having an ethylene content of 0.5 to 8% by weight, a butene-1 content of 0.5 to 8% by weight and a melting temperature of 155 to 120°C is melted in an extruder at a cylinder temperature of 180 to 250°C, extruded as a pipe by a pipe die, and sized in a cooling sleeve at a temperature of 5 to 90°C while reducing the pressure inside the cooling sleeve, to prepare a biocompatible material of the invention in the form of a pipe having a thickness of 0.3 to 3 mm.

(6) T-die molding: A propylene homopolymer having a tacticity corresponding to a heptane extraction residue of 88 to 90% and a melting temperature of 160 to 165°C, an ethylene-propylene copolymer having an ethylene content of 0.5 to 8% by weight and a melting temperature of 155 to 120°C, or an ethylene-propylene-butene-1 copolymer having an ethylene content of 0.5 to 8% by weight, a butene-1 content of 0.5 to 8% by weight and a melting temperature of 155 to 120°C is melted in an extruder at a cylinder temperature of 180 to 300°C, extruded from a slot, and cooled with cooling rolls cooled with water at a temperature of 5 to 90°C to prepare a biocompatible material of the invention in the form of a film or sheet having a thickness of 0.01 to 3 mm.

The biocompatible material of the invention thus molded is sufficiently stable and the blood compatibility is not lowered over a temperature range of -20 to 80°C, and therefore can be used at an ambient temperature at which the material is used, e.g., -10 to 40°C.

In the molding, an anti-oxidizing agent, such as tetrakis[methylene(3,5-di-t-butyl-4-hydroxyhydrocinnamate)] methane, and a scavenger of residues (e.g., chlorine) of the catalysts used in the synthesis of the polymeric material, such as hydrotalcite ($Mg_{4.5}Al_2(OH)_{18}CO_8 \cdot 3.5H_2O$) may be added to the polymeric material.

The biocompatible material of the invention has the specified micro-phase separation structure at the surface as described above, and therefore the multiple adsorption of plasma proteins onto the surface is suppressed, avoiding the activation of blood platelets to prevent the coagulation of blood. Accordingly, when a medical instrument having at least a surface portion to contact blood in use is made by using the biocompatible material of the invention such that the blood contacting surface of the instrument is provided by the surface of the micro-phase separation structure of the biocompatible material, a medical instrument having a high biocompatibility (blood compatibility) can be obtained. Such medical instruments include those to contact blood, those to dwell in a blood vessel, and those to dwell in a body cavity, and more specifically artificial organs such as artificial blood vessels, extracorporeal circulation circuits for artificial dialyzators and lungs, catheters such as PTCA catheters and intravenous catheters, blood bags, and blood administration sets for the administration of blood preparations.

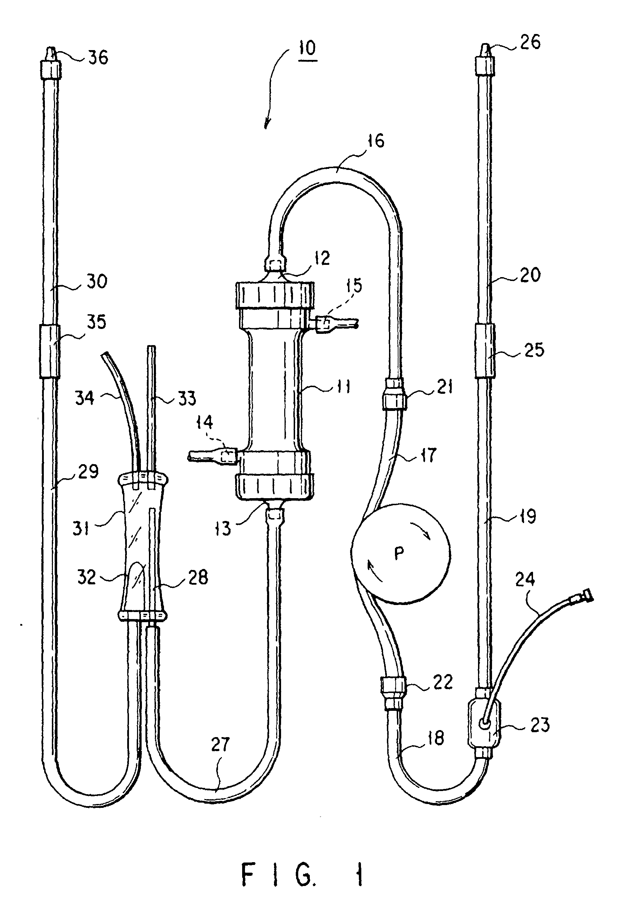

FIG. 1 shows an extracorporeal circulation circuit 10 for blood made by using the biocompatible material of the invention. An artificial dialyzator 11 is incorporated in the circuit 10. In the dialyzator 11, a large number of blood dialyzing tubes (not shown) are accommodated. Blood entering the dialyzator 11 at a blood inlet port 12 flows within each tube and flows out at a blood outlet port 13. The blood is dialyzed with a dialyzing liquid entered at dialyzing liquid inlet port 14 and flowing outside each dialyzing tube while the blood passes through the inside of each dialyzing tube. The dialyzing liquid flows out through a dialyzing liquid outlet port 15. To the blood inlet port 12 is connected a blood tube 16, which is, in turn, connected to a blood tube 17 through a connector 21. The blood tube 17 is connected to a blood tube 18 through connector 22. The blood tube 18 is connected to a blood tube 19 through a connector 23. The blood tube 19 is connected a blood tube 20 through a connector 25. The blood tube 17 is squeezed by a pump P so as to adjust the flow rate of blood. To the connector 23 is connected a tube 24 through which a drug such as a blood anti-coagulant may be administered. To the tip end of the tube 20 is mounted an attachment 26 for attaching a needle (not shown) which is to be pierced into a blood vessel of the subject to send the blood to be dialyzed to the dialysis tube system.

To the blood outlet port 13 is connected a dialyzed blood tube 27, which is connected to a dialyzed blood chamber 31 through a smaller tube 28 inserted into the chamber 31. Tubes 33 and 34 to be connected to a pressure detection device (not shown) are connected to the upper end portion of the chamber 31. The dialyzed blood flowing out of the tube 28 in the chamber 31 passes through a blood filter 32 provided in the chamber 31, and is finally returned to the subject through a dialyzed blood tube 29 and a dialyzed blood tube 30 connected to the tube 29 through a connector 35. To the tip end of the tube 30 is connected an attachment 36 for attaching a needle (not shown) which is to be pierced into a blood vessel of the subject to return the dialyzed blood to the subject.

In the blood circulation circuit described above, the tubes 16 to 20 and tubes 27 to 30, as well as the blood chamber 31 may be made of the biocompatible material of the present invention. Instead of the dialyzator 11, an artificial lung may be used.

FIG. 2 shows a blood bag system 40. The blood bag system 40 has a blood bag body 41, whose peripheral portion is heat-sealed. A blood administering tube 43 communicates with the inside of the bag body 41. A piercing needle for insertion into the blood vessel is connected to the tip end of the tube 43 through a hub 47. Blood discharging ports 44 and 45 communicate with the inside of the bag body 41. Excluding the piercing needle 46 and the hub 47, the bag body 41, the tube 43, and the ports 44 and 45 can be made of the biocompatible material of the present invention.

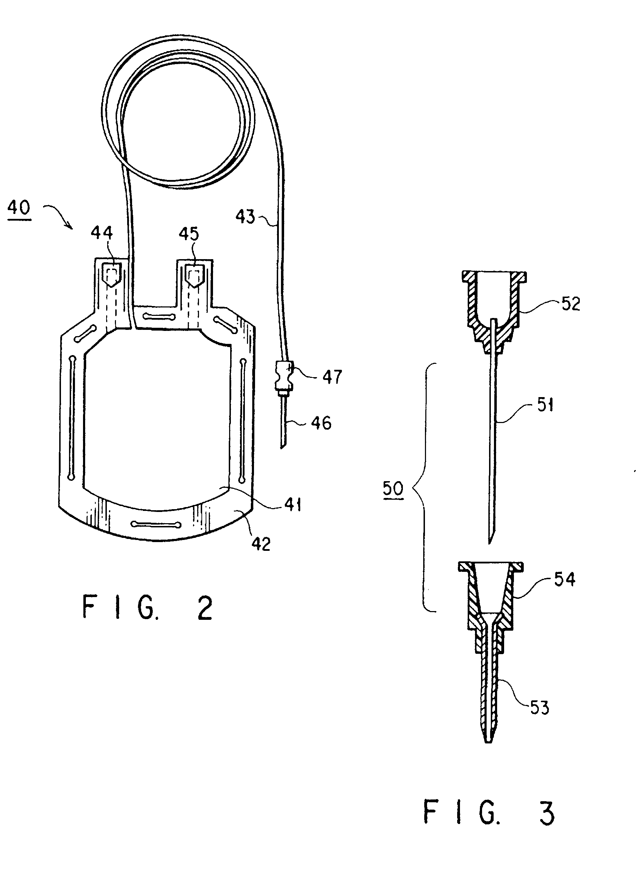

FIG. 3 shows an intravenous catheter device 50. The device shown is the combination of a cannula 51 for insertion into a blood vessel, attached to a hub 52, with a catheter for dwelling in the blood vessel, attached to a hub 54. The cannula 51 is pierced into the blood vessel through the catheter dwelling in the blood vessel such that it slightly protrudes from the distal end of the catheter 53. In the catheter device 50 described, the catheter 53 can be made of the biocompatible material of the present invention.

The present invention will now be described by way of Examples which follow.

Preparation of Polymer Pellets:

Preparation A

100 parts by weight of a propylene homopolymer having a melt flow rate (230°C-21.18N) of 8 g/10 min., a melting temperature of 165°C and a tacticity corresponding to a heptane extraction residue of about 97% by weight was added with 0.1 parts by weight of tetrakis[methylene(3,5-di-t-butyl-4-hydroxyhydrocinnamate)]methane and 0.05 parts by weight of hydrotalcite were added, and was uniformly mixed in a Henschel mixer. The resultant mixture was placed in an extruder and melted and kneaded at a temperature of 220C. The material was extruded, and the extrudate was immediately cut with a cutter rotating at a high speed to prepare polypropylene pellets. These pellets is named PP1.

Preparation B

Polymer pellets were prepared in the same manner as in Preparation A, except that an ethylene-propylene copolymer having a melt flow rate (230°C-21.18N) of 8 g/10 min., a melting temperature of 160°C, a tacticity corresponding to a heptane extraction residue of about 94% by weight, and an ethylene content of 0.3% by weight was used as a starting polymer. These pellets are named PP2 pellets.

Preparation C

Polymer pellets were prepared in the same manner as in Preparation A, except that an ethylene-propylene copolymer having a melt flow rate (230°C-21.18N) of 8 g/10 min., a melting temperature of 143°C, a tacticity corresponding to a heptane extraction residue of about 94% by weight, and an ethylene content of 2.5% by weight was used as a starting polymer. These pellets are named PP3 pellets.

Preparation D

Polymer pellets were prepared in the same manner as in Preparation A, except that an ethylene-propylene-butene-1 copolymer having a melt flow rate (230°C-21.18N) of 8 g/10 min., a melting temperature of 135°C, a tacticity corresponding to a heptane extraction residue of about 60% by weight, an ethylene content of 3% by weight and a butene-1 content of 1.5% by weight was used as a starting polymer. These pellets are named PP4 pellets.

Preparation E

Polymer pellets were prepared in the same manner as in Preparation A, except that an ethylene-propylene-butene-1 copolymer having a melt flow rate (230°C-21.18N) of 5 g/10 min., a melting temperature of 130°C, a tacticity corresponding to a heptane extraction residue of about 55% by weight, an ethylene content of 3.5% by weight and a butene-1 content of 2.5% by weight was used as a starting polymer. These pellets are named PP5 pellets.

Preparation F

Polymer pellets were prepared in the same manner as in Preparation A, except that an ethylene-propylene-butene-1 copolymer having a melt flow rate (230°C-21.18N) of 5 g/10 min., a melting temperature of 128°C, a tacticity corresponding to a heptane extraction residue of about 50% by weight, an ethylene content of 4.5% by weight and a butene-1 content of 2.5% by weight was used as a starting polymer. These pellets are named PP6 pellets.

Preparation G

Polymer pellets were prepared in the same manner as in Preparation A, except that an ethylene-propylene copolymer having a melt flow rate (230°C-21.18N) of 2 g/10 min., a melting temperature of 138°C, a tacticity corresponding to a heptane extraction residue of about 70% by weight and an ethylene content of 3.5% by weight was used as a starting polymer. These pellets are named PP7 pellets.

Preparation H

Polymer pellets were prepared in the same manner as in Preparation A, except that a propylene homopolymer having a melt flow rate (230°C-21.18N) of 2 g/10 min., a melting temperature of 165°C and a tacticity corresponding to a heptane extraction residue of about 97% by weight was used as a starting polymer. These pellets are named PP8 pellets.

Preparation I

Polymer pellets were prepared in the same manner as in Preparation A, except that an ethylene-propylene copolymer having a melt flow rate (230°C-21.18N) of 2 g/10 min., a melting temperature of 143°C, a tacticity corresponding to a heptane extraction residue of about 70% by weight and an ethylene content of 2.5% was used as a starting polymer. These pellets are named PP9 pellets.

Preparation J

Polymer pellets were prepared in the same manner as in Preparation A, except that an ethylene-propylene-butene-1 copolymer having a melt flow rate (230°C-21.18N) of 2 g/10 min., a melting temperature of 135°C, a tacticity corresponding to a heptane extraction residue of about 65% by weight, an ethylene content of 3% by weight and a butene-1 content of 1.5% by weight was used as a starting polymer. These pellets are named PP10 pellets.

Preparation K

Polymer pellets were prepared in the same manner as in Preparation A, except that an ethylene-propylene-butene-1 copolymer having a melt flow rate (230°C-21.18N) of 2 g/10 min., a melting temperature of 128°C, a tacticity corresponding to a heptane extraction residue of about 55% by weight, an ethylene content of 4.5% by weight and a butene-1 content of 2.5% by weight was used as a starting polymer. These pellets are named PP11 pellets.

Preparation of Biocompatible Materials:

Example 1

A biocompatible material in the form of a sheet having a thickness of 0.5 mm was prepared using the PP1 pellets obtained in Preparation A, by compression molding method under the molding conditions such that the melting temperature was 200°C, the melting time was 3 minutes, the cooling temperature was 50°C, and the cooling time was 3 minutes.

Example 2 - 12

Sheets having a thickness of 0.5 mm were prepared in the same manner as in Example 1, except that the pellets used, and the cooling temperature and time were changed as indicated in Table 1 below.

Example 13

A biocompatible material in the form of a hollow article (tube) having a thickness of 1 mm was prepared using the PP8 pellets obtained in Preparation H, by blow molding method under the molding conditions such that the cylinder temperature of the extruder was 210°C, the temperature of the mold-cooling water was 25°C, and the cooling time was 3 minutes.

Examples 14 - 17

Tubes having a thickness of 1 mm were prepared in the same manner as in Example 13, except that the pellets used, the cylinder temperature and the mold-cooling water temperature were changed as indicated in Table 2 below.

Example 18

A biocompatible material in the form of a sheet having a thickness of 0.5 mm was prepared using the PP8 pellets obtained in Preparation H, by T-die molding method under the molding conditions such that the cylinder temperature of the extruder was 230°C, and the roll temperature was 70°C.

Example 19 - 22

Sheets having a thickness of 0.5 mm were prepared in the same manner as in Example 18, except that the pellets used, the cylinder temperature and the roll temperature were changed as indicated in Table 3 below.

Example 23

A biocompatible material in the form of an hollow article (tube) having a thickness of 0.8 mm was prepared using the PP1 pellets obtained in Preparation A, by injection molding method under the molding conditions such that the cylinder temperature of the extruder was 230°C, and the mold-cooling water temperature was 30°C.

Example 24 - 28

Tubes having a thickness of 0.8 mm were prepared in the same manner as in Example 23, except that the pellets used, the cylinder temperature and the mold-cooling water temperature were changed as indicated in Table 4 below.

The biocompatible materials prepared in Examples 1 to 28 were measured for the surface crystallinity, the bulk crystallinity, the long period of repeated thickness of crystalline and amorphous phases, and the crystallite thickness by the methods previously described. Further, the amount of plasma proteins (albumin and fibrinogen) adsorbed and the concentration of free $Ca^{2+}$ in the cytoplasm were measured on these biocompatible materials. The results are also shown in Tables 1 to 4. The measurements on the amount of plasma proteins adsorbed and the free $Ca^{2+}$ concentration were conducted as follows. Plasma Proteins Adsorption Test: Rabbit serum albumin (RSA), 0.072g, and rabbit plasma fibrinogen (RPF), 0.0048g, were dissolved in 0.04 M phosphate buffer solutions (0.04 M-PBS), respectively. Each of the protein solutions was placed in a dialyzer provided with a dialyzing membrane having a cut-off molecular weight of 12,000-14,000, and dialyzed against one liter of 0.04 M-PBS at 0°C. The PBS was exchanged every one hour, and the dialysis was conducted for a total of 6 hours. After the dialysis, each protein solution was lyophilized.

Then, the lyophilized RSA was dissolved in a 0.04 M-PBS to provide an RSA solution having an RSA concentration of 0.045 g/dl. On the other hand, the lyophilized RPF was dissolved in a 0.04 M-PBS to provide an RPF solution having an RPF concentration of 0.003 g/dl. While 16 ml of each of the protein solutions was maintained at 37°C, a polymer sample having a surface area of about 12 $cm^2$ was immersed in each solution for an hour to adsorb the protein. Thereafter, the protein solution was substituted with about 300 ml of 0.04 M-PBS. Then, the polymer sample was dipped in a 1% by weight aqueous solution of sodium dodecylsulfate to peel off the protein adsorbed on the polymer sample. The protein solution thus obtained was quantitatively measured for the amount of the protein after incubation at 60°C for an hour by Bicichoninic acid method using MICRO BCA PROTEIN ASSAY REAGENT available from PIERCE. The quantitative measurement was conducted at a wavelength of 562 nm using MICROPLATE READER MODEL 550 available from BIO-RAD.

The Cytoplasmic free calcium concentration in platelets: Blood was collected from the femoral artery of a male rabbit generally anesthetized with nembutal by the conventional method, such that the ratio of 3.8% aqueous solution of sodium citrate to blood collected became 1 : 9 (volume). The collected blood was centrifuged at 1,000 rpm for 15 minutes to provide platelet-rich plasma (PRP). 3 ml of the PRP was centrifuged at 1,500 rpm for 10 minutes to give platelet pellets, which were slowly re-suspended in 1 ml of Hanks solution to adjust the concentration of the platelets to about $3 \times 10^8$/ml. FURA 2-AM available from WAKO PURE CHEMICALS INDUSTRIES, LTD., Japan was mixed with physiological saline at a ratio of 1 : 10 by volume. This mixture, 3 ml, was mixed with 2 ml of the platelet solution, and was incubated at 37C for 45 to 60 minutes. The centrifugation at 1,200 rpm and the re-suspension of the platelets in Hanks solution were repeated twice to remove the excessive FURA 2-AM, and the concentration of the platelets were finally adjusted to about $3 \times 10^8$/ml. The platelet suspension loaded with FURA 2-AM, 2 ml, was introduced into a mini blood bag prepared from the biocompatible sheet of the invention. The bag was shielded from light by aluminum foil, and was incubated at 37°C for an hour while being shaken. One milliliter of the platelet suspension recovered from the bag was charged into a spitz tube shielded from light by aluminum foil, added with 6 µl of a 2% aqueous solution of calcium chloride, and was incubated at 37°C for 5 minutes. Then, the thus incubated suspension, 400 µl, was placed in a microcubette provided with a stirrer, and was set in a spectrophotoflurorimeter CAF-110 available from JASCO CO., LTD. Japan, and the ratio (R) of the fluorescence intensity at an excitation wavelength of 340 nm to the fluorescence intensity at an excitation wavelength of 380 nm was recorded. Thereafter, a 10% aqueous solution of TRITON-X was added to solubilize the platelets, and the fluorescence intensity ($R_{max}$) was recorded. Then, 10 µl of a 30 mM aqueous solution of EGTA (pH > 8.3) was added with a microsyringe, and the fluorescence intensity ($R_{min}$) was recorded. The intracellular concentration of free $Ca^{2+}$ ($[Ca^{2+}]_i$) was calculated by the following equation:

$$[Ca^{2}+]_i = Kd[(R - R_{min})/(R_{max} - R)] \times (Sf_2/Sb_2)$$

where Kd is 224 nM which is the dissociation constant of $Ca^{2+}$, and $Sf_2/Sb_2$ is the ratio of the fluorescence intensity, $R_{min}$, at an excitation wavelength of 380 nm to the fluorescence intensity, $R_{max}$, at an excitation wavelength of 380 nm.

These procedures mentioned above can show the changes in platelet cytoplasm which occur at the very initial stage when the platelets initiate reaction (activation) in response to the stimulations from the surroundings, and are described by, for example, N. Yui, et al., Artificial Organs, Vol. 20 (No. 2), 103-108 (1996). Note that highly activated platelet cells show a free $Ca^{2+}$ concentration of several thousands nM, while non-activated platelets show a free $Ca^{2+}$ concentration of about 200 nM.

Table 1

| | Pellets | Cooling Temperature (°C) | Cooling Time (min.) | Degree of Surface Crystallinity (%) | Degree of Bulk Crystallinity (%) | Long Period (nm) | Crystallite Thickness (nm) | RSA Adsorbed ($\mu g/cm^2$) | RPF Adsorbed ($\mu g/cm^2$) | Free $Ca^{2+}$ conc. (nM) |
|---|---|---|---|---|---|---|---|---|---|---|
| Example 1 | PP1 | 50 | 3 | 61 | 69 | 11.7 | 8.1 | 0.42 | 0.23 | |
| Example 2 | PP2 | 50 | 3 | 62 | 70 | 11.6 | 8.1 | 0.37 | 0.23 | 1183 |
| Example 3 | PP3 | 50 | 3 | 57 | 68 | 10.9 | 7.4 | 0.3 | 0.28 | |
| Example 4 | PP7 | 50 | 3 | 55 | 67 | 10.7 | 7.2 | 0.23 | 0.14 | 398 |
| Example 5 | PP4 | 50 | 3 | 53 | 66 | 10.7 | 7 | 0.33 | 0.13 | 939 |
| Example 6 | PP5 | 50 | 3 | 49 | 65 | 11 | 7.2 | 0.42 | 0.31 | |
| Example 7 | PP6 | 50 | 3 | 45 | 63 | 11 | 7 | 0.66 | 0.31 | |

(continued)

Table 1

| | Pellets | Cooling Temperature (°C) | Cooling Time (min.) | Degree of Surface Crystallinity (%) | Degree of Bulk Crystallinity (%) | Long Period (nm) | Crystallite Thickness (nm) | RSA Adsorbed ($\mu g/cm^2$) | RPF Adsorbed ($\mu g/cm^2$) | Free $Ca^{2+}$ conc. (nM) |
|---|---|---|---|---|---|---|---|---|---|---|
| Example 8 | PP2 | 30 | 3 | 55 | 70 | 10.9 | 7.6 | 0.18 | 0.14 | |
| Example 9 | PP1 | 30 | 3 | 56 | 69 | 11.4 | 7.9 | 0.24 | 0.33 | |
| Example 10 | PP3 | 70 | 5 | 54 | 69 | 11.4 | 7.9 | 0.15 | 0.23 | |
| Example 11 | PP5 | 70 | 5 | 53 | 65 | 11.5 | 7.5 | 0.15 | 0.31 | |
| Example 12 | PP6 | 80 | 10 | 54 | 64 | 12 | 7.7 | 0.18 | 0.23 | |

Table 2

|  | Pellets | Cylinder Temperature (°C) | Mold-Cooling Water temperature (°C) | Degree of Surface Crystallinity (%) | RSA Adsorbed ($\mu g/cm^2$) | RPF Adsorbed ($\mu g/cm^2$) |
|---|---|---|---|---|---|---|
| Example 13 | PP8 | 210 | 25 | 57 | 0.38 | 0.27 |
| Example 14 | PP8 | 210 | 40 | 59 | 0.36 | 0.3 |
| Example 15 | PP9 | 210 | 25 | 55 | 0.22 | 0.13 |
| Example 16 | PP10 | 200 | 30 | 52 | 0.24 | 0.15 |
| Example 17 | PP11 | 200 | 50 | 53 | 0.21 | 0.12 |

Table 3

|  | Pellets | Cylinder Temperature (°C) | Roll temperature (°C) | Degree of Surface Crystallinity (%) | RSA Adsorbed ($\mu g/cm^2$) | RPF Adsorbed ($\mu g/cm^2$) |
|---|---|---|---|---|---|---|
| Example 18 | PP8 | 230 | 70 | 63 | 0.48 | 0.3 |
| Example 19 | PP8 | 230 | 30 | 55 | 0.2 | 0.13 |
| Example 20 | PP9 | 230 | 60 | 60 | 0.4 | 0.21 |
| Example 21 | PP10 | 210 | 60 | 57 | 0.28 | 0.24 |
| Example 22 | PP11 | 210 | 70 | 53 | 0.29 | 0.14 |

Table 4

| | Pellets | Cylinder Temperature (°C) | Mold-Cooling Water temperature (°C) | Degree of Surface Crystallinity (%) | RSA Adsorbed (µg/cm$^2$) | RPF Adsorbed (µg/cm$^2$) |
|---|---|---|---|---|---|---|
| Example 23 | PP8 | 230 | 30 | 52 | 0.2 | 0.11 |
| Example 24 | PP8 | 230 | 50 | 57 | 0.18 | 0.1 |
| Example 25 | PP8 | 230 | 70 | 64 | 0.31 | 0.19 |
| Example 26 | PP9 | 230 | 40 | 54 | 0.19 | 0.09 |
| Example 27 | PP10 | 230 | 50 | 56 | 0.21 | 0.13 |
| Example 28 | PP11 | 230 | 60 | 55 | 0.18 | 0.12 |

**Claims**

1. A biocompatible material which comprises at least a polymer selected from an ethylene homopolymer, a propylene homopolymer, an ethylene-propylene copolymer, an ethylene-propylene-butene-1 copolymer, an ethylene-hexene-1 copolymer, an ethylene-octene-1 copolymer, an ethylene-butene-1-hexene-1 copolymer, an ethylene-butene-1-octene-1 copolymer, an ethylene-hexene-1-octene-1 copolymer and an ethylene-butene-1-hexene-1-octene-1 copolymer, and which has a surface region of micro-phase separation structure comprising crystalline and amorphous phases, wherein said biocompatible material has a degree of bulk crystallinity of 40 to 70%, said surface region has a degree of crystallinity of 40 to 70%, said micro-phase separation structure has a long period of 8 to 20 nm in the repeated thickness, and said crystalline phase has crystallite thickness of 4 to 14 nm.

2. A medical instrument having a portion to contact blood, **characterized in that** at least a part of said blood contacting portion is made of a biocompatible material as defined in claim 1.

**Patentansprüche**

1. Biokompatibler Werkstoff, der mindestens aus einem Polymer besteht, ausgewählt aus einem Ethylen-Homopolymer, einem Propylen-Homopolymer, einem Ethylen-Propylen-Copolymer, einem Ethylen-Propylen-Buten-1-Copolymer, einem Ethylen-Hexen-1-Copolymer, einem Ethylen-Octen-1-Copolymer, einem Ethylen-Buten-1-Hexen-1-Copolymer, einem Ethylen-Buten-1-Octen-1-Copolymer, einem Ethylen-Hexen-1-Octen-1-Copolymer und einem Ethylen-Buten-1-Hexen-1-Octen-1-Copolymer, und einen Oberflächenbereich aus einer Mikrophasen-Trennstruktur mit kristallinen und amorphen Phasen aufweist, wobei der biokompatible Werkstoff einen Volumenkristallinitätsgrad von 40 bis 70% aufweist, der Oberflächenbereich einen Kristallinitätsgrad von 40 bis 70% hat, die besagte Mikrophasen-Trennstruktur eine Periode von 8 bis 20 nm in der wiederholten Dicke hat und die kristalline Phase eine Kristallitdicke von 4 bis 14 nm aufweist.

2. Medizinisches Instrument mit einem Abschnitt, der mit Blut in Kontakt kommt, **dadurch gekennzeichnet, dass** zumindest ein Teil des Abschnitts, der mit Blut in Kontakt kommt, aus einem biokompatiblen Werkstoff nach Anspruch 1 besteht.

**Revendications**

1. Matériau biocompatible qui comprend au moins un polymère choisi parmi un homopolymère de l'éthylène, un homopolymère du propylène, un copolymère éthylène-propylène, un copolymère éthylène-propylène-but-1-ène, un copolymère éthylène-hex-1-ène, un copolymère éthylène-oct-1-ène, un copolymère éthylène-but-1-ène-hex-1-ène, un copolymère éthylène-but-1-ène-oct-1-ène, un copolymère éthylène-hex-1-ène-oct-1-ène et un copolymère éthylène-but-1-ène-hex-1-ène-oct-1-ène, et qui a une région de surface de structure à séparation de microphases comprenant des phases cristalline et amorphe, où ledit matériau biocompatible a un degré de cristallinité globale de 40 à 70 %, ladite région de surface a un degré de cristallinité de 40 à 70 %, ladite structure à séparation de microphases a une longue période de 8 à 20 nm dans l'épaisseur répétée, et ladite phase cristalline a une épaisseur de cristallite de 4 à 14 nm.

2. Instrument médical ayant une portion destinée à venir en contact avec le sang **caractérisé en ce qu'**au moins une partie de ladite portion venant en contact avec le sang est constituée par un matériau biocompatible selon la revendication 1.

F I G. 1

F I G. 2

F I G. 3